# EUROPEAN PATENT APPLICATION

(11) **EP 3 392 345 A1**
(43) Date of publication of application: **24.10.2018**
(21) Application number: 17000678.7
(22) Date of filing: 22.04.2017
(51) Int. Cl.: C12Q 1/68, A61K 31/7088

(54) **BIOMARKER FOR SMALL CELL LUNG CANCER THERAPY**

(71) Applicant: Mologen AG, 14195 Berlin (DE)
(72) Inventor: The designation of the inventor has not yet been filed
(74) Representative: von Renesse, Dorothea

(57) **Abstract**

A TLR-9 agonist provided for use in treating small cell lung cancer (SCLC) in a subject in need thereof, wherein the subject to be treated has an elevated level of activated B cells and/or is diagnosed with chronic obstructive pulmonary disease (COPD).

## Description

### FIELD OF THE INVENTION

The present invention relates generally to the identification of small cell lung cancer (SCLC) patients as to whether they will respond to specific therapies. More particularly, the invention relates to a TLR-9 agonist, preferably Lefitolimod, for use in treating SCLC, a method for predicting whether a subject will respond to treatment with the TLR-9 agonist, and a pharmaceutical composition comprising said TLR-9 agonist. The invention particularily relates to the TLR-9 agonist Lefitolimod.

### BACKGROUND OF THE INVENTION

Biomarkers are substances found in blood or other body fluids or in tissues. For example, biomarkers are receptors on cells that signal the presence or absence of a condition or disease, like cancer for instance. One example of biomarkers are predictive biomarkers. Most often, they are used to assess the probability that a patient will respond or benefit from a particular treatment.

Treatment of cancer with anti-cancer drugs, in particular chemotherapy and especially targeted treatments, usually involve the use of very aggressive drugs and thus often cause severe adverse effects. In order to avoid unnecessary treatments there is a constant need for means allowing to assess whether a patient will have a benefit from a particular treatment.

With regards to the fact that therapies become more and more target specific, the role of biomarkers will even increase. Biomarkers will help to individualise therapeutic approaches and to ease the way to individualised oncology. Also the possibility to distinguish a responder to a specific therapy from a non-responder will help to prevent that patients will be subject to unnecessary treatments.

In order to reduce side effects of chemotherapeutics many attempts have been undertaken, e.g. minimizing the dosage of chemotherapeutics by combining them with immune activating agents. One approach is the use of immune activating DNA (hereinafter also "polydeoxyribonucleotides" or, simply "polynucleotides").

So-called unmethylated CG nucleotide sequences ("CG dinucleotide") have been shown to activate the immune system very effectively (Krieg AM, Yi AK, Matson S, Waldschmidt TJ, Bishop GA, Teasdale R, Koretzky GA, Klinman DM; "CpG motifs in bacterial DNA trigger direct B-cell activation" Nature 1995 Apr 6 374:6522 546-9). Those sequences are derived from bacteria. EP 1 196 178 A1 discloses covalently closed circular DNA with partially self-complementary sequences resulting in a polynucleotide having a double stranded stem with single stranded loops at both ends (giving a dumbell-shaped structure) comprising the unmethylated CG motifs.

The combination of the dumbbell-shaped polynucleotides of EP 1 196 178 A1 with chemotherapeutics to treat cancerous diseases has been proposed by EP 1 776 124 A1. It is disclosed that patients who were treated with these polynucleotides subsequently received a chemotherapeutic treatment. Therewith the amount of chemotherapeutic could be reduced. The combination of these compounds with immunotherapy, namely the combination with checkpoint inhibitors is known from WO2017/042336 A1.

Small cell lung cancer (SCLC) accounts for approximately 15 % of bronchogenic carcinomas. At the time of diagnosis, approximately 30 % of patients with SCLC have tumors confined to the hemithorax of origin, the mediastinum, or the supraclavicular lymph nodes. These patients are designated as having limited-stage SCLC. Patients with tumors that have spread beyond the supraclavicular areas are said to have extensive-stage SCLC.

Small cell lung cancer is more responsive to chemotherapy and radiotherapy than other cell types of lung cancer; however, a cure is difficult to achieve because SCLC has a greater tendency to be widely disseminated by the time of diagnosis. Without treatment, SCLC has the most aggressive clinical course of any type of pulmonary tumor, with median survival from diagnosis of only 2 to 4 months. Despite improvements in diagnosis and therapy made during the past 25 years, the current prognosis for patients with SCLC is unsatisfactory.

At present, treatment options for SCLC patients are determined by histology, stage, and general health and comorbidities of the patient. Investigations of patients with suspected SCLC focus on confirming the diagnosis and determining the extent of the disease.

A method for assessing whether a patient suffering from cancer will respond to a treatment with the dumbbell-shaped polynucleotides of EP 1 196 178 A1 has been proposed by the international patent application WO 2014/191222 A1. This approach is based on determining the frequency of the activated natural killer T ("NKT") in the patient to be treated. However, this document is silent about the possibility to use this information - or any other biomarker - to assess whether an SCLC patient will benefit from the treatment of TLR-9 agonists such as the polynucleotides of EP 1 196 178 A1.

It is thus an objective of the present invention to provide a method for assessing whether a patient suffering from SCLC will benefit from a treatment with a TLR-9 agonist, in particular with the dumbbell-shaped polydeoxyribonucleotides of EP 1 196 178 A1, even more particular with the polydeoxyribonucleotide Lefitolimod. Furthermore it is an objective of the invention to provide a TLR-9 agonist for use in treating of a patient with SCLC and a pharmaceutical composition comprising said TLR-9 agonist.

### SUMMARY OF THE INVENTION

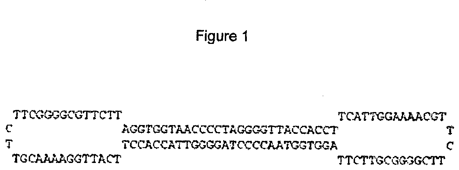

This objective is solved by the use of the TLR-9 agonist according to claim 1. The inventors have found that an SCLC patient benefits from a treatment with a TLR-9 agonist, in particular with a polydeoxyribonucleotide as disclosed in EP 1 196 178, most preferred the immunomodulatory polydeoxyribonucleotide known to the skilled person under the INN Lefitolimod (CAS registry number 1548439-51-5; also known as "MGN1703"; cf. Fig. 1), when the subject to be treated has one or both of the following characteristics
i. a ratio of activated B cells with respect to the total number of CD 19 positive cells of less than or equal to 20 %, preferably of less than or equal to 18 %, more preferably of less than or equal to 17 %, most preferably less than or equal to 15.4 %, and/or
ii. a diagnosed chronic obstructive pulmonary disease (COPD).

Activated B cells can be determined by the expression of the activation marker cluster of differentiation (CD)86. The ratio of activated B cells is given with respect to the total number of CD 19 positive cells.

According to the invention, thus the ratio of the activated B cells to the total number of CD19 positive cells given above and/or the fact that the SCLC patient suffers from COPD can serve as biomarkers to predict if an SCLC patient will respond to a treatment with a TLR-9 agonist, in particular with Lefitolimod. In particular, with these markers it can be assessed if the patient most likely will benefit from a treatment with a TLR-9 with regard to his overall survival (OS). This in particular applies when the TLR-9 agonist (in particular Lefitolimod) is used in a switch-maintenance therapy with a chemotherapy or radiotherapy. As a chemotherapy platinum-based agents are especially preferred.

These biomarkers can also support the assessment of the quality of the response of the patient to the treatment with a TLR-9 agonist. Thus, in yet a further aspect the invention relates to a method for predicting the overall survival, partial response (PR) or complete response (CR) in a subject suffering from SCLC, wherein the subject is to be treated with a TLR-9 agonist. Therewith the invention also provides a method for discriminating among subjects suffering from SCLC between a responder and a non-responder to treatment with a TLR-9 agonist. Hence the invention helps avoiding treatment of patients without clinical benefit to the patient.

Furthermore the invention relates to an especially suitable administration/application and/or dosing scheme for treating SCLC patients with a TLR-9 agonist, in particular with Lefitolimod. A pharmaceutical composition comprising Lefitolimod and which can be used in the methods of the invention is suggested as well.

### DETAILED DESCRIPTION OF THE INVENTION

A TLR-9 agonist is provided, preferably Lefitolimod, for use in treating small cell lung cancer (SCLC) in a subject in need thereof, wherein the subject to be treated has one or more of the following characteristics
i. a ratio of activated B cells with respect to the total number of CD19 positive cells of less than or equal to 20 %, preferably of less than or equal to 18 %, more preferably of less than or equal to 17 %, most preferably less than or equal to 15.4 %, and/or
ii. a diagnosed chronic obstructive pulmonary disease (COPD).

Activated B cells can be determined by the expression of the activation marker cluster of differentiation (CD)86. The ratio of activated B cells is given with respect to the total number of CD19 positive cells.

These characteristics of the subject are preferably determined before beginning the treatment of the subject with the TLR-9 agonist (baseline). They preferably can serve as a biomarker to predict whether the subject to be treated will respond, in particular benefit, from the treatment with the TLR-9 agonist.

"Toll-like receptors" (TLRs) are part of the innate immune system of vertebrates. TLRs are a family of specialized immune receptors that induce protective immune responses when they detect highly conserved pathogen-related molecular patterns, such as proteins, lipid structures, saccharidic structures, and certain nucleic acids. Synthetic agonists for several TLRs, including TLR-3, TLR-4, TLR-7, TLR-8, and TLR-9, have been or are being developed for the treatment of cancer, generally with the intention to activate the immune system in the presence of tumours.

TLR-9 recognizes the presence of unmethylated CG-containing DNA sequences, which are typically found in bacteria, but practically never in human genomic DNA. Thus, polydeoxynucleotides comprising unmethylated CG-containing DNA sequences ("CG motifs") have been designed as synthetic TLR-9 agonists. Especially advantageous dumbbell-shaped TLR-9 agonists are disclosed in EP 1 196 178 A1. Other preferred immune activating polydeoxyribonucleotides are known from WO 2012/085291 A1. The disclosure of these documents with respect to the polydeoxyribonucleotides most preferred therein is fully incoporated herein as references.

In a preferred embodiment of the invention the subject suffers form extensive-stage SCLC (hereinafter also designated as "extensive SCLC" or "ED"). ED is defined as SCLC that has spread beyond the supraclavicular areas. It is too widespread to be encompassed within a tolerable radiation therapy port. Patients with pleural effusion, massive pulmonary tumor, contralateral supraclavicular nodes, and any distant metastasis are included in this definition of extensive-stage SCLC.

The invention, however, also relates to limited-stage SCLC (designated hereinafter also as "limited SCLC" or "LD"). LD is a form of SCLC which is confined to the hemithorax of origin, the mediastinum, or the supraclavicular lymph nodes.

Established guidelines exist for the diagnosis of SCLC. They include pathological, histological and/or cytological methods. Guidelines are provided for example by the European Society for Medical Oncology (Früh. M et al. "Small-cell lung cancer (SCLC): ESMO Clinical Practice Guidelines for diagnosos, treatment and follow-up". Annals of Oncology 24 (Suppl. 6) vi99-vi105. 2013).

Chronic obstructive pulmonary disease (COPD) is an obstructive lung disease characterized by long-term poor airflow. The main symptoms include shortness of breath and cough with sputum production. COPD is a progressive disease which means that it typically worsens over time.

Globally, it is estimated that about 3 million deaths were caused by the disease in 2015 (that is, 5% of all deaths globally in that year). COPD is not curable, but treatment can relieve symptoms, improve quality of life and reduce the risk of death.

Various methods are available to the person skilled in the art to diagnose COPD. Established methods include lung (pulmonary) function tests, such as spirometry. Spirometry can detect COPD even before symptoms arise. Other lung function tests include measurement of lung volumes, diffusing capacity and pulse oximetry. Other possibilities are Chest X-ray, CT scan or arterial blood gas analysis.

In a particular embodiment of the invention, the subject to be treated with the TLR-9 agonist has received a cancer treatment before the treatment with the TLR-9 agonist. This earlier cancer treatment can be a treatment with at least one chemotherapeutic or a radiotherapy. In a particularly preferred embodiment, the first cancer treatment is a chemotherapy.

Preferably, the earlier cancer treatment is a therapy with a platinum-based chemotherapeutic. Platinum-based chemotherapeutics are substances (also called simply "platins"), which are characterized by a coordination complex with platinum. It is supposed that their action is based on a crosslinking of DNA. According to the invention the platinum-based chemotherapeutic preferably can be selected form the group consiting of cisplatin, carboplatin, oxaliplatin, nedaplatin, triplatin tetranitrate, phenanthriplatin, picoplatin and satraplatin or a mixture hereof. The group consiting of cisplatin, carboplatin, oxaliplatin and nedaplatin is preferred. Even more preferred platin-based chemotherapeutics are cisplatin and carboplatin; cisplatin is most preferred. Also a treatment with a combination of more than one platinum-based chemotherapeutic is possible.

In another preferred embodiment of the invention, the subject to be treated has received earlier a platinum-based chemotherapeutic in combination with a further anti-cancer agent. This combination therapy preferably includes cisplatin or carboplatin as the platinum-based chemotherapeutic. The further anti-cancer agent preferably is selected from the group consisting of etoposide, irinotecan, gemcitabine and topotecan. Most preferably, the combination therapy is a treatment with etoposide-cisplatin, irinotecan-cisplatin, gemcitabine-carboplatin and topotecan-cisplatin.

Unless otherwise explicitly mentioned, in the context of the present invention, the term "platinum-based chemotherapy" includes both, the treatment of the subject with one or more platinum-based chemotherapeutics and the treatment with a combination of one or more platinum-based chemotherapeutics and at least one further anti-cancer agent.

According to a preferred embodiment of the invention the subject to be treated has received the platinum-based chemotherapeutics as the first-line therapy. This means, that the subject to be treated with the TLR-9 agonist according to the invention has not received a treatment with an anti-cancer chemotherapeutic prior to the platinum-based treatment. Preferably the subject has completed, prior to the treatment with the TLR-9 agonist, at least one cycle, preferably at least four cycles.

"Radiotherapy" refers to therapy using ionizing radiation. Radiotherapy is commonly applied to the cancerous tumor because of its ability to control cell growth. Ionizing radiation works by damaging the DNA of cancerous tissue leading to cellular death. Application of radiotherapy may also be termed irradiation.

As outlined above, in one preferred embodiment of the present invention, the subject to be treated has completed at least one treatment cycle of a first anti-cancer therapy, in particular a platinum-based chemotherapy, preferably at least four treatment cycles, most preferred four treatment cycles. A treatment cycle refers to a course of treatment that is repeated on a regular schedule with periods of rest in between. For example, a treatment given for one week followed by three weeks of rest is one treatment cycle. Depending on the chemotherapeutics or combinations thereof as well as the condition of the subject to be treated the duration of a treatment cycle can vary.

It is also included that a subject receives one platinum-based chemotherapeutic in one cycle and the same or another platinum-based chemotherapeutic in another cycle, or a combination of platinum-based chemotherapeutics in one cycle and the same or another combination of platinum-based chemotherapeutics in another cycle.

Preferably, the subject to be treated according to the invention has responded to an earlier anti-cancer treatment, in particular the subject has shown a partial response (PR) or a complete response (CR) to an earlier anti-cancer treatment, in particular to a platinum-based chemotherapy, before the treatment with the TLR-9 agonist. Hence, in this embodiment of the invention, a first therapy has resulted in a PR or CR of the SCLC patient. A subject that has responded to a treatment (either with a PR or a CR) may be referred to as a "responder".

With respect to the response of a subject to a treatment it may further be discriminated between a progressive disease (PD) and stable disease (SD).

A partial response is understood as a decrease in the size of a tumor, or in the extent of cancer in the body. A complete response refers to a condition that the symptoms caused by the tumour have disappeared, preferably but not necessarily the tumour has disappeared. This does, however, not mean that the cancer has been cured.

The response of a patient to cancer treatment can be assessed by the so called "RECIST guidelines", which are well established criteria accepted by the scientific community and regulatory authorities (Eisenhauer et al.: "New response evaluation criteria in solid tumours: Revised RECIST guideline (version 1.1)" European Journal of Cancer 45 (2009) 228-247).

In according with the RECIST guidelines (version 1.1) imaging-based evaluation is preferred over clinical examination unless the lesion(s) being evaluated can only be assessed by clinical examination. Preferred imaging-based methods are computed tomography (CT) and/or magnetic resonance imaging (MRI). CT and/or MRI scans are performed of the thorax of the patient including the upper part of abdomen. On the basis of the RECIST evaluation, and in a preferred embodiment of the invention, the response of a patient to a anti-cancer treatment can be qualified as follows:
Complete Response: Disappearance of all target lesions. Any pathological lymph nodes (whether target or non-target) must have reduction in short axis to < 10 mm.
Partial Response: At least a 30 % decrease in the sum of diameters of target lesions, taking as reference the baseline sum diameters.
Progressive Disease: At least a 20 % increase in the sum of diameters of target lesions, taking as reference the smallest sum on study. In addition to the relative increase of 20%, the sum must also demonstrate an absolute increase of at least 5 mm. The appearance of 1 or more new lesions is also considered progression.
Stable disease: Neither sufficient shrinkage to qualify for PR nor sufficient increase to qualify for progressive disease, taking as reference the smallest sum diameters while on study.

Alternatively, a response of a patient to a treatment can also be assessed by the "immune-related response criteria" (irRC). This evaluation approach was developed in 2009 to capture additional response patterns, based on the modified WHO criteria using bidimensional tumor measurements of target lesions (Wolchok JD, Hoos A, O'Day S, et al.. "Guidelines for the evaluation of immune therapy activity in solid tumors: immune-related response criteria." Clin Cancer Res. 2009;15(23):7412-20.)

According to the invention, the RECIST guidelines are preferably applied to qualify a response of a patient to an anti-cancer treatment, in particular as PR or CR. These criteria are also preferably applied to qualify a patient as being a possible responder or non-responder to the treatment of the TLR-9 agonists, in particular to Lefitolimod.

When the subject to be treated with the TLR-9 agonist has received a first anti-cancer treatment, the biomarkers as of the invention - the activated B-cells and the presence/absence of COPD - preferably are determined after the completion of the first anti-cancer treatment and before the treatment with the TLR-9 agonist; however in any event before the first administration of the TLR-9 agonist.

According to one preferred embodiment of the invention the TLR-9 agonist is used as a monotherapy. The term "monotherapy" refers to the use of a drug as the single active pharmaceutical ingredient (API) to treat a particular disorder or disease. Thus, in a monotherapy the drug is administered without the combination with another drug. However, the term monotherapy inludes that another drug substance may be administered before or after administration of the drug used in a monotherapy.

In one preferred embodiment of the invention, the TLR-9 agonist, preferably Lefitolimod, is used as a monotherapy. Before and/or after administration of the TLR-9 agonist the subject preferably receives a chemotherapeutic, in particular the subject receives a platinum-based chemotherapy.

In yet another preferred embodiment of the invention the TLR-9 agonist, in particular Lefitolimod, is used in a switch maintenance therapy. Switch maintenance according to the invention means that an anti-cancer agent is used after completion of at least one treatment cycle with a different first anti-cancer agent. According to a preferred embodiment of the invention, at first a platinum-based chemotherapy is completed, preferably at least four cycles, and then the TLR-9 agonist is introduced into the treatment regime.

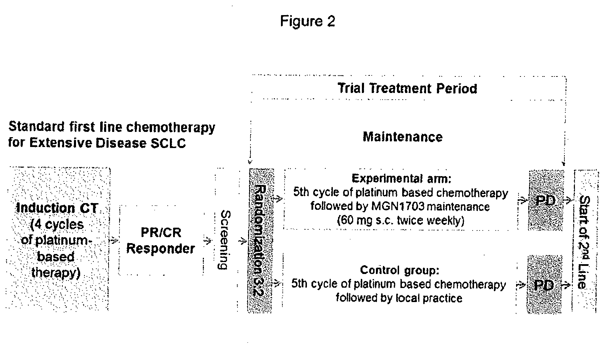

In a particularity preferred embodiment the TLR-9 agonist, especially Lefitolimod, is first administered after completion of four treatment cycles of a platinum-based chemotherapy. In a 5th treatment cycle of the chemotherapy, the TLR-9 agonist is administered. It is preferred to administer the TLR-9 agonist at least 1, preferably at least 2, more preferably at least 3 and even more preferred at least 7 days after the administration of the chemotherapeutic used in the chemotherapy. Most preferred is the administration of the TLR-9 agonist in the week of the 5th cycle when there is no administration of the chemotherapeutics. The same regime can be repeated in a 6th cycle of the chemotherapy treatment. This regime (study design) is depicted in Fig. 2.

In a preferred embodiment of the invention the TLR-9 agonist is administered to the patient at least twice. The minimum interval between these two administration is preferably 48. It can be administered at least twice a week, preferably twice a week.

It is preferred that the TLR-9 agonist is given in an amount of 10 to 200 mg, preferred 40 to 100 mg, most preferred 60 mg per day. The daily dose is preferably splitted into two administrations (most preferred of 30 mg each). This allows the administration into two different sites of the patient's body. Preferably the daily dose is given twice a week. The TLR-9 agonist most preferred is given by injection, most preferably subcutaneously.

In is advantageous that the TLR-9 agonist is given at different sites of the patient's body. It is preferred that the application regime comprises a left and right upper arm of the subject, a left and right thigh of the subject and/or a left and right para-umbilical region of the subject. Hence it is preferred that the TLR-9 agonist is administered in an alternating rotational scheme of at least two different application sites.

This above decribed preferred administration and dosing regimes in particular apply to Lefitolimod and, preferably, to the treatment of extensive-stage SCLC with Lefitolimod.

The treatment according to the invention can also form part of a combined-modality therapy. A combined-modality therapy is a therapeutic approach when a patient is treated with two or more of different treatment modalities, such as surgery, radiation therapy, immunotherapy or any type of medication (e.g. chemotherapeutics or biologics, such as antibodies). The combination of platinum-based chemotherapeutics with cranial irradiation, and furtheron, the administration of a TLR-9 agonist according to the present invention can be of particular advantage.

The TLR-9 agonist according to the invention preferably comprises a polydeoxyribonucleotide comprising at least one unmethylated CG dinucleotide, preferably at least two CG, wherein C is deoxycytidine and G is deoxyguanosine. The at least one CG dinucleotide can be part of the sequence N¹N²CGN³N⁴, wherein N¹N² is AA, TT, GG, GT, GA or AT and N³N⁴ is CT, TT or GG and C is deoxycytidine, G is deoxyguanosine, A is deoxyadenosine, and T is deoxythymidine.

In a preferred embodiment the TLR-9 agonist comprises at least one nucleotide in L-configuration. This is preferably located within the terminal five nucleotides of at least one end of the polydeoxyribonucleotide. In this embodiment the polydeoxyribonucleotide preferably comprises at least 20 nucleotides, more preferably 20 to 25 nucleotides.

The polydeoxyribonucleotide comprises preferably a double-stranded stem and two single-stranded loops and forms the shape of a dumbbell. The polydeoxyribonucleotide preferably is covalently closed. It is advantegous if least one CG dinucleotide is located in one or each of the single-stranded loops.

A "stem" according to the present disclosure shall be understood as a DNA double strand formed by base pairing either within the same DNA molecule (which is then partially self-complementary) or within different DNA molecules (which are partially or completely complementary). Intramolecular base pairing designates base pairing within the same molecules, and base pairing between different DNA molecules is termed as intermolecular base-pairing.

A "loop" within the meaning of the present disclosure shall be understood as an unpaired, single-stranded region either within or at the end of a stem structure.

A "dumbbell-shape" describes a polynucleotide which comprises a double-stranded stem (base pairing within the same polynucleotide) and two single stranded loops at both ends of the double-stranded stem.

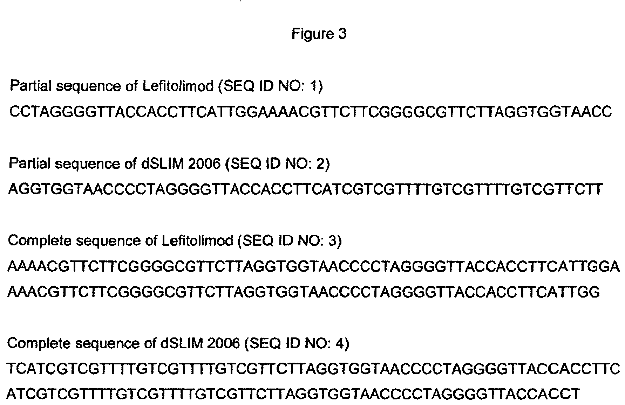

In these dumbell-shaped polynucleotides preferably all nucleotides are in D-configuration. Furthermore it is preferred that the polydeoxyribonucleotide comprises at least 50, preferably at least 80, most preferably 116 nucleotides. The nucleotide sequences of two preferred representatives of this type of TLR-9 agonists are depicted in Fig. 3.

The most preferred preferred TLR-agonist for use according to the invention is Lefitolimod (SEQ ID NO:3).

In yet another embodiment of the invention a method is provided for predicting, whether a subject suffering from SCLC, in particular suffering from extended-stage SCLC, will respond to a treatment with a TLR-9 agonist, in particular with Lefitolimod, by determining before treatment with the TLR-9 agonist
i. a ratio of activated B cells with respect to the total number of CD19 positive cells, and/or
ii. whether the subject has been diagnosed with COPD.

The activated B-cells and the total number of CD19 positive cells are measured in a blood sample (whole blood) of the subject to be treated.

In yet another embodiment of the invention a method is provided for discriminating among subjects suffering from SCLC, in particular suffering from extended-stage SCLC, between a responder and a non-responder to treatment with a TLR-9 agonist, in particular with Lefitolimod, by determining before treatment with the TLR-9 agonist
i. a ratio of activated B cells with respect to the total number of CD19 positive cells, and/or
ii. whether the subject has been diagnosed with COPD.

It is particularily preferred to assess, by determining the biomarkers as decribed above, whether the responder will have a better overall survival (OS) than the non-responder. "Overall survival" is defined as the time interval starting with the treatment and lasting until death (=event) or until the date of last information available from the patient.

Hence, in yet another preferred embodiment, the invention relates to activated B cell for use as a biomarker for predicting a response to treatment with a TLR-9 agonist, preferably Lefitolimod, in a subject suffering from cancer, in particular from SCLC.

"Biomarkers" are substances found in blood or other body fluids or in tissues. For example, biomarkers are receptors on cells that signal the presence or absence of a condition or disease, like cancer for instance. One example of biomarkers are predictive biomarkers. Most often, they are used to assess the probability that a patient will respond or benefit from a particular treatment.

In a further embodiment of the invention a pharmaceutical composition comprising Lefitolimod, is provided, which is suitable for the treatment of SCLC. This composition comprises 1 mg/ml to 30 mg/ml, preferably 10 mg/ml to 20 mg/ml, more preferably 15 mg/ml of Lefitolimod in PBS for use in treating SCLC, wherein the PBS has a pH of pH 6 to 8, in particular 7.0 to 7.5, and comprises
∘ 6 mg/ml to 12 mg/ml, preferably 8.8 mg/ml of sodium chloride,
∘ 0.1 mg/ml to 0.3 mg/ml, preferably 0.22 mg/ml of potassium chloride
∘ 0.1 mg/ml to 0.3 mg/ml, preferably 0.22 mg/ml of potassium dihydrogen phosphate and
∘ 1.0 mg/ml to 1.5 mg/ml, preferably 1.265 mg/ml of disodium hydrogen phosphate.

### BRIEF DESCRIPTION OF THE DRAWINGS

- FIG. 1: Sequence and structure of Lefitolimod (MGN1703)
- FIG. 2: Study Design
- FIG. 3: Partial and complete sequences of the dumbbell-shaped TLR-9 agonists Lefitolimod (MGN1703) and dSLIM 2006. Partial sequences are used for the synthesis of the complete sequences.
- FIG. 4: Kaplan-Meier plot (overall survival vs. time to event in days) for the subgroup of patients with a ratio of activated B cells ≤ 15.42 %
- FIG. 5: Kaplan-Meier plot (overall survival vs. time to event in days) for the subgroup of patients with diagnosed COPD

### SPECIFIC EMBODIMENTS

1. A TLR-9 agonist, preferably Lefitolimod, for use in treating small cell lung cancer (SCLC) in a subject in need thereof, wherein the subject to be treated has one or more of the following characteristics:
   i. a ratio of CD86 positive B cells with respect to the total number of CD19 positive cells of less than or equal to 20 %, preferably of less than or equal to 18 %, more preferably of less than or equal to 17 %, most preferably less than or equal to 15.4 %, and/or
   ii. a diagnosed chronic obstructive pulmonary disease (COPD).
2. The TLR-9 agonist for use according to embodiment 1, wherein the small cell lung cancer is limited small cell lung cancer or extensive small cell lung cancer, preferably extensive small cell lung cancer.
3. The TLR-9 agonist for use according to embodiment 2, wherein the limited or extensive small cell lung cancer is identified pathologically, histologically and/or cytologically.
4. The TLR-9 agonist for use according to any of the preceding embodiments, wherein the subject has received a first cancer treatment before treatment with the TLR-9 agonist.
5. The TLR-9 agonist for use according to embodiment 4, wherein the first cancer treatment is treatment with a chemotherapeutic or radiotherapy.
6. The TLR-9 agonist for use according to embodiment 5, wherein the treatment with a chemotherapeutic is a first-line therapy with a platinum-based chemotherapeutic, preferably with a platinum-based combination chemotherapeutic.
7. The TLR-9 agonist for use according to embodiment 6, wherein the subject has completed at least one cycle of first-line therapy, preferably four cycles.
8. The TLR-9 agonist for use according to embodiment 7, wherein the cycle has a length of more than one week.
9. The TLR-9 agonist for use according to embodiment 8, wherein the first-line therapy is administered within the first week of each cycle.
10. The TLR-9 agonist for use according to any of embodiments 5 to 9, wherein the platinum-based chemotherapy comprises a first anti-cancer agent and a second anti-cancer agent and the first anti-cancer agent is selected from the group consisting of etoposide, irinotecan, gemcitabine and topotecan, and the second anti-cancer agent is selected from the group consisting of cisplatin and carboplatin.
11. The TLR-9 agonist according to embodiment 10, wherein the platinum-based chemotherapy is selected from the group comprising etoposide-cisplatin, irinotecan-cisplatin, gemcitabine-carboplatin and topotecan-cisplatin.
12. The TLR-9 agonist for use according to any of embodiments 6 to 10, wherein the subject has received no other prior chemotherapy.
13. The TLR-9 agonist for use according to any of embodiments 4 to 12, wherein the subject has achieved partial response (PR) or complete response (CR) before treatment with the TLR-9 agonist.
14. The TLR-9 agonist for use according to embodiment 13, wherein PR or CR is assessed using CT or MRI scan.
15. The TLR-9 agonist for use according to any of the preceding embodiments, wherein the TLR-9 agonist is used as a monotherapy.
16. The TLR-9 agonist for use according to any of the preceding embodiments, wherein the TLR-9 agonist is used in a switch maintenance therapy.
17. The TLR-9 agonist for use according to embodiment 16, wherein the switch maintenance therapy comprises administration of a first-line therapy with a platinum-based chemotherapeutic followed by administration of the TLR-9 agonist.
18. The TLR-9 agonist according to embodiment 17, wherein the subject has completed four cycles of a first-line therapy with a platinum-based chemotherapeutic and receives a fifth and/or sixth cycle of a first-line therapy with a platinum-based chemotherapeutic and the platinum-based chemotherapeutic is only administered during a first week of each cycle.
19. The TLR-9 agonist according to embodiment 18, wherein the TLR-9 agonist is administered starting in the week of the fifth and/or sixth cycle when there is no administration of the chemotherapeutic.
20. The TLR-9 agonist for use according to any of the preceding embodiments, wherein the TLR-9 agonist is used in a combined-modality therapy.
21. The TLR-9 agonist for use according to embodiment 20, wherein the combined-modality therapy comprises a further treatment selected from the group consisiting of surgery, radiotherapy, and medication, wherein medication is administration of a chemotherapeutic or a biologic.
22. The TLR-9 agonist for use according to embodiment 20 or 21, wherein a first-line therapy with a platinum-based chemotherapeutic is combined with cranial irradiation.
23. The TLR-9 agonist for use according to any of the preceding embodiments, wherein the TLR-9 agonist is administered at least twice.
24. The TLR-9 agonist for use according to embodiment 23, wherein there is a minimum interval of 48 hours between two administrations.
25. The TLR-9 agonist for use according to any of the preceding embodiments, wherein the TLR-9 agonist is administered at least twice a week, preferably twice a week.
26. The TLR-9 agonist for use according to any of the preceding embodiments, wherein 10 to 200 mg of the TLR-9 agonist are administered, preferably 40 to 100 mg, most preferably 60 mg.
27. The TLR-9 agonist for use according to any of the preceding embodiments, wherein 60 mg of the TLR-9 agonist are administered twice a week.
28. The TLR-9 agonist for use according to any of the preceding embodiments, wherein 30 mg of the TLR-9 agonist are administered at two application sites twice a week.
29. The TLR-9 agonist for use according to any of the preceding embodiments, wherein the subject has completed four cycles of a first-line therapy with a platinum-based chemotherapeutic and receives a fifth cycle of a first-line therapy with a platinum-based chemotherapeutic followed by administration of the TLR-9 agonist, preferably Lefitolimod, wherein during the fifth cycle the platinum-based chemotherapeutic is administered during a first week of the fifth cycle, and the TLR-9 agonist, preferably Lefitolimod, is administered during a second and third week of the fifth cycle twice a week.
30. The TLR-9 agonist for use according to embodiment 27, wherein the subject receives a sixth cycle of a first-line therapy with a platinum-based chemotherapy followed by administration of the TLR-9 agonist, preferably Lefitolimod, wherein during the sixth cycle the platinum-based chemotherapeutic is administered during a first week of the sixth cycle, and the TLR-9 agonist, preferably Lefitolimod, is administered during a second and third week of the sixth cycle twice a week.
31. The TLR-9 agonist for use according to any of the preceding embodiments, wherein the TLR-9 agonist is administered by injection, preferably subcutaneously.
32. The TLR-9 agonist for use according to any of the preceding embodiments, wherein the TLR-9 agonist, preferably Lefitolimod, is administered twice a week subcutaneously as switch maintenance therapy in a subject with extensive small cell lung cancer and wherein the subject achieved at least a partial response following first-line therapy with a platinum-based chemotherapeutic.
33. The TLR-9 agonist for use according to any of embodiments 29 to 32, wherein 30 mg of the TLR-9 agonist are administered at two application sites twice a week.
34. The TLR-9 agonist for use according to any of the preceding embodiments, wherein each administration comprises administration at two application sites.
35. The TLR-9 agonist for use according to embodiment 34, wherein the application sites comprise a left and right upper arm of a subject, a left and right thigh of a subject and a left and right para-umbilical region of a subject.
36. The TLR-9 agonist for use according to embodiment 34 or 35, wherein the application sites are used alternately in a rotational scheme.
37. The TLR-9 agonist for use according to any of the preceding embodiments, wherein the TLR-9 agonist comprises a polydeoxyribonucleotide comprising at least one unmethylated CG dinucleotide, wherein C is deoxycytidine and G is deoxyguanosine.
38. The TLR-9 agonist for use according to embodiment 37, wherein the at least one CG dinucleotide is part of a sequence N¹N²CGN³N⁴, wherein N¹N² is AA, TT, GG, GT, GA or AT and N³N⁴ is CT, TT or GG and C is deoxycytidine, G is deoxyguanosine, A is deoxyadenosine, and T is deoxythymidine.
39. The TLR-9 agonist for use according to embodiment 37 or 38, wherein the polydeoxyribonucleotide comprises at least one nucleotide in L-configuration.
40. The TLR-9 agonist for use according to any of embodiments 37 to 39, wherein the polydeoxyribonucleotide comprises at least 20 nucleotides, preferably 20 to 25 nucleotides.
41. The TLR-9 agonist for use according to embodiment 39 or 40, wherein the at least one nucleotide in L-configuration is comprised within the terminal five nucleotides of at least one end of the polydeoxyribonucleotide.
42. The TLR-9 agonist for use according to any of embodiments 39 to 41, wherein at least three consecutive deoxyguanosines in D-configuration are located within the terminal six nucleotides of at least one end of the polydeoxyribonucleotide.
43. The TLR-9 agonist for use according to any of embodiments 37 to 42, wherein the polydeoxyribonucleotide comprises at least two CG dinucleotides.
44. The TLR-9 agonist for use according to embodiment 43, wherein at least three consecutive deoxyguanosines are located in between two CG dinucleotides.
45. The TLR-9 agonist for use according to embodiment 43, wherein at least five nucleotides are located between two CG dinucleotides, excluding deoxyguanosine.
46. The TLR-9 agonist according to any of embodiments 37 to 45, wherein the polydeoxyribonucleotide is single-stranded and/or partially or completely double-stranded.
47. The TLR-9 agonist for use according to embodiment 46, wherein the at least one CG dinucleotide is located in the single- and/or double-stranded region of the polydeoxyribonucleotide.
48. The TLR-9 agonist for use according to any of embodiments 37 to 47, wherein the polydeoxyribonucleotide comprises a double-stranded stem and at least one single-stranded loop.
49. The TLR-9 agonist for use according to embodiment 48, wherein the polydeoxyribonucleotide comprises two single-stranded loops and forms the shape of a dumbbell.
50. The TLR-9 agonist for use according to embodiment 48 or 49, wherein all nucleotides are in D-configuration.
51. The TLR-9 agonist for use according to any of embodiments 48 to 50, wherein the polydeoxyribonucleotide comprises at least 50, preferably at least 80, most preferably 116 nucleotides.
52. The TLR-9 agonist for use according to any of embodiments 48 to 51, wherein the polydeoxyribonucleotide comprises at most 200, preferably at most 150, most preferably 116 nucleotides.
53. The TLR-9 agonist for use according to any of embodiments 48 to 52, wherein the at least one CG dinucleotide is located in
   a) one or each of the single-stranded loops, preferably in each of the single-stranded loops or
   b) the double-stranded stem, or
   c) one or each of the single-stranded loops and in the double-stranded stem.
54. The TLR-9 agonist for use according to any of embodiments 48 to 53, wherein the polydeoxyribonucleotide comprises a double-stranded stem and at least one single-stranded loop and the at least one CG dinucleotide is located in the single-stranded loop.
55. The TLR-9 agonist for use according to any of embodiments 48 to 54, wherein the polydeoxyribonucleotide comprises a double-stranded stem, two single-stranded loops and forms the shape of a dumbbell and at least one CG dinucleotide is located in each of the single-stranded loops.
56. The TLR-9 agonist for use according to embodiment 55, wherein three CG dinucleotides are located in each of the single-stranded loops.
57. The TLR-9 agonist for use according to any of embodiments 48 to 56, wherein one or each of the single-stranded loops comprises at least 20 nucleotides, preferably consists of 30 nucleotides.
58. The TLR-9 agonist for use according to embodiment 49, wherein each of the single-stranded loops consists of 30 nucleotides and is of identical sequence.
59. The TLR-9 agonist for use according to any of embodiments 48 to 58, wherein the double-stranded stem comprises at least 15, preferably at least 20, most preferably consists of 28 base pairs.
60. The TLR-9 agonist for use according to any of embodiments 48 to 59, wherein the double-stranded stem comprises at most 90, preferably at most 60, most preferably consists of 28 base pairs.
61. The TLR-9 agonist for use according to embodiment 49, wherein each of the single-stranded loops consists of 30 nucleotides, the double-stranded stem consists of 28 base pairs and three CG dinucleotides are located in each of the single-stranded loops.
62. The TLR-9 agonist for use according to embodiment 49, wherein the TLR-9 agonist is covalently closed and consists of twice a partially hybridized sequence of SEQ ID NO: 1
63. The TLR-9 agonist for use according to embodiment 49, wherein the TLR-9 agonist is covalently closed and consists of twice a partially hybridized sequence of SEQ ID NO: 2
64. The TLR-9 agonist for use according to embodiment 49, wherein the TLR-9 agonist is covalently closed and consists of the sequence of SEQ ID NO: 3.
65. The TLR-9 agonist for use according to embodiment 49, wherein the TLR-9 agonist is covalently closed and consists of the sequence of SEQ ID NO: 4.
66. The TLR-9 agonist for use according to any of embodiments 37 to 65, wherein at least one nucleotide is modified with a functional group selected from the group comprising carboxyl, amine, amide, aldimine, ketal, acetal, ester, ether, disulfide, thiol and aldehyde groups.
67. The TLR-9 agonist for use according to embodiment 66, wherein the modified nucleotide is linked to a compound selected from the group comprising peptides, proteins, carbohydrates, antibodies, lipids, micelles, vesicles, synthetic molecules, polymers, micro projectiles, metal particles, nanoparticles, and a solid phase.
68. Method of treatment of small cell lung cancer (SCLC) in a subject comprising administering to the subject an effective amount of a TLR-9 agonist, wherein the subject to be treated has one or more of the following characteristics
   i. a ratio of CD86 positive B cells with respect to the total number of CD19 positive cells of less than or equal to 20 %, preferably of less than or equal to 18 %, more preferably of less than or equal to 17 %, most preferably less than or equal to 15.4 %, and/or
   ii. a diagnosed chronic obstructive pulmonary disease (COPD).

### SYNTHESIS OF A TLR-9 AGONIST AS SHOWN FOR MGN1703 (LEFITOLIMOD)

5'-phosphorylated oligonucleotides with the sequence CCTAGGGGTT ACCACCTTCA TTGGAAAACG TTCTTCGGGG CGTTCTTAGG TGGTAACC (TIB-Molbiol, Berlin), as shown in SEQ ID NO: 1, were heated to a temperature of 90° C for 5 minutes and subsequently cooled on ice to enable development of a stem-loop structure. Self-complementary overhangs of such oligonucleotides were ligated with a final concentration of 1 µg/µl DNA in the presence of T4 DNA ligase (0.1 U/µg oligonucleotide) at 37° C for 24 hours. The product was obtained following phenol extraction and subsequent extraction with chloroform as well as isopropanol precipitation in the present of MgCl2 (final concentration 10 mM) and NaAc (final concentration 300 mM), and after centrifugation and suspension in water.

In order to remove endotoxin contamination, the ligation product was subject to subsequent anion exchange chromatography (carrier substance: LiChrospher DMAE, Merck Darmstadt; 0-1M NaCl in 50 mM Na3PO4) and concentrated by isopropanol precipitation. For in vivo experiments, this method is carried out in sterile conditions and the end product is suspended in sterile PBS.

FIG. 1 shows the structure of the MGN1703 (i.e. Lefitolimod) sequence (SEQ ID NO: 3), which is a covalently closed dumbbell-shaped polydeoxyribonucleotide of 116 nucleotides linked by phosphodiester bonds. It consists of two single-stranded loops of 30 bases each, and a double-stranded stem of 28 base pairs. The sequence is non-coding but contains a total of six CG dinucleotides with 3 CG dinucleotides in each loop.

FIG. 3 shows the sequences of SEQ ID NO: 1 and SEQ ID NO: 3 (partial and complete sequence of Lefitolimod (MGN1703)) as well as the partial (SEQ ID NO: 2) and complete (SEQ ID NO: 4) sequence of an alternative dumbbell-shaped TLR-9 agonist (dSLIM 2006).

### EXAMPLE: CLINICAL STUDY "IMPULSE"

### 1. Synopsis

The data according the present invention result from an open-label, randomized clinical study of switch maintenance therapy with the immunomodulator MGN1703 (synonymous: Lefitolimod) in patients with extensive SCLC, who have undergone four cycles of first-line chemotherapy with a platinum-based chemotherapeutic.

Patients with extensive SCLC, confirmed by a pathologist on the basis of a histology of SCLC or mixed histology of SCLC, were eligible for the study if they have had four cycles of first-line therapy with a platinum-based chemotherapeutic and have confirmed partial response (abbreviation: PR) or complete response (abbreviation:CR) at the end of the fourth cycle. A cytological diagnosis of SCLC, confirmed by a pathologist, was acceptable if histology could not be obtained for any reason. Eastern Cooperative Oncology Group (abbreviation: ECOG) performance status had to be 0 or 1 (Oken MM, Creech RH, Tormey DC, et al. (1982). "Toxicity and response criteria of the Eastern Cooperative Oncology Group". Am. J. Clin. Oncol. 5 (6): 649-55). Screening has been started after the last application in the fourth cycle of platinum-based chemotherapy.

Eligible patients have been randomized (in a 3:2 ratio) to receive a fifth cycle of chemotherapy followed by treatment with MGN1703 (Arm A) or a fifth cycle of chemotherapy followed by local standard care (Arm B; there are no global standard treatment options for these patients). After randomization, patients will be in the study until death or for a maximum of 2 years.

The Treatment Phase has been started after randomization. During this phase, patients have received a fifth cycle of platinum-based chemotherapy (scheduled in line with local care). In the following, patients randomized to Arm A (the experimental arm) have received MGN1703 administered s.c. at 2 application sites twice a week, starting in the week of the fifth cycle when there is no chemotherapy administration. Patients in Arm B (the control arm) have received any standard of care according to local clinical practice, which may include for instance continuation of the same platinum-based chemotherapeutic, start of a new maintenance regimen, or best supportive care without chemotherapy.

According to local practice, investigators could choose to:
- Stop first-line therapy with a platinum-based chemotherapeutic once the patient has completed four cycles of platinum-based chemotherapy; in this case patients in Arm A have only received MGN1703 and patients in Arm B have received local standard care.
- Give an additional sixth cycle of a platinum-based chemotherapeutic. Similarly to the fifth cycle, this is followed by treatment with MGN1703 administered s.c. at 2 application sites twice a week (Arm A (the experimental arm)) or followed by local standard care (Arm B (control arm)).
The Treatment Phase had a maximum of 2 years. After stopping the Treatment Phase, all patients entered a post-treatment Surveillance Phase, which stopped at a maximum of 2 years after randomization. During this phase, survival status and cancer therapy was assessed every 12 weeks.

To be eligible for study entry patients had in particular to satisfy the following criteria:
Extensive SCLC confirmed by a pathologist, on the basis of histology of SCLC or mixed histology of SCLC, or a cytological diagnosis if histology cannot be obtained;
Completion of four cycles of first-line therapy with a platinum-based chemotherapeutic and no other prior chemotherapy;
Documented evidence of tumour response (PR or CR) as assessed by the investigator at the end of the fourth cycle of the first-line therapy with the platinum-based chemotherapeutic using CT or MRI scan.

In total, 102 patients suffering from SCLC who had previously been treated for four cycles in a standard first-line therapy with a platinum-based chemotherapeutic were selected for the study.

After a randomization of the patients was performed, 62 patients received a fifth and/or sixth cycle of a platinum-based chemotherapeutic followed by MGN1703 maintenance (60 mg s.c. per dose of the TLR-9 agonist MGN1703 (i.e. Lefitolimod) twice weekly) and 40 patients received a fifth and/or sixth cycle of a platinum-based chemotherapeutic followed by any standard of care according to local clinical practice.

Every 8 +/- 2 weeks of treatment, all patients were examined for tumour progression. Treatment was continued for each patient until tumour progression was assessed.

Prior to the first application of MGN1703 or control at visit 1 (at baseline) blood samples of all patients were collected.
An overview of the study design is given in FIG. 2.

### 2. Sample handling

Whole blood (10 mL) for Fluorescence-activated cell sorting (FACS) analysis was collected in Streck Cyto-Chex® BCT tubes. Within 2 hours after sampling the blood samples were shipped to the analytical laboratory. Samples were analyzed within 24 hours after blood draw. According to an established protocol, samples were shipped and stored at room temperature before analysis.

### 3. Analytical Methods - FACS analysis

The ratio of activated B cells with respect to the total number of CD19 positive cells was evaluated. The ratios were documented for each sample of each patient.

B cells were identified by the following combination of cluster of differentiation (CD) molecules: CD45+ / CD19+.
Activated B cells were identified by the following combination of CD molecules: CD45+ / CD 19+ / CD86+.

FACS analyses were performed according to established protocols known to the skilled person. Whole blood samples were lysed and stained with fluorescence labeled antibodies. Table 1 shows the reagents used for flow cytometry.

**Table 1: List of flow cytometry reagents**

| Specificity | Label | Supplier | Reference |
|---|---|---|---|
| CD169 | PE | eBioscience | 12-1699 |
| CD86 (PC5.5) | PC5.5 / PC5 | Beckman Coulter | B30647 |
| CD14 | APC | Beckman Coulter | IM2580U |
| CD19 | PacBlue | Beckman Coulter | A86355 |
| CD45 | KrOranqe | Beckman Coulter | A96416 |

Isotype controls were used for every experiment to discriminate specific from nonspecific binding. CD45 was used as leukocyte marker.

Samples were run on a 10-colour Navios flow cytometer and analysed using Navios Software (all from Beckman Coulter).

The detailed staining protocol is as follows:

Cells were stained with the following combination of monoclonal antibodies: Anti CD169-PE, Anti CD86-PC5.5, Anti CD14-APC, Anti CD19-PacBlue, Anti CD45-KrOrange.

B cells were gated as CD19 positive cells. Within the B cell population, CD86 was used as activation marker.

### 4. Results

The following parameters were determined and evaluated. A correlation of treatment outcome (overall survival, OS) with the parameters measured at baseline has been analyzed.

### Ratio of activated B cells

### Diagnosis of COPD

In a first step univariate Cox regression models have been analyzed to identify parameters that might have an effect on outcome (OS). Significance level for parameters to be considered in the following steps was 0.2. Correlations of the parameters identified in this step have been analyzed to provide additional information for interpretation of the following multivariate analyses.

Multivariate Cox regression models have been analyzed including "ratio of activated B cells" and "diagnosis of COPD" as parameters identified in the previous step. Backward selection have been applied to eliminate parameters that do not have an effect on outcome (OS) in the multivariate analysis. Significance level for removing parameters from the model was 0.1.

A Cox regression allows estimating the effect of parameter(s) without any consideration of the hazard function. The resulting hazard ratio from such a calculation should be below 1 and related to a significant p value being below 0.05. Otherwise the observed effect would not be related to the applied TLR-9 agonist. Those criteria applied both to "ratio of activated B cells" and "diagnosis of COPD".

### Activated B cells

For the ratio of activated B cells a "derived cut-off value" was calculated as follows:
a. For each possible dichotomization of patients, a Cox proportional hazard model for OS was analyzed and respective hazard ratios with 95 % Cls were calculated.
b. The "derived cut-off value" is defined as the value where the most significant correlation of the subgroup with OS is observed in the model (smallest p-value).

The "derived cut-off value" was calculated as 15.42 %.

The following results were obtained for the ratio of activated B cells (Table 2):

**Table 2: Results for a ratio of activated B cells ≤ 15.42 %**

| Derived cut-off value | MGN1703 Median (days) | Control Median (days) | Hazard ratio | 95 % Wald CI |
|---|---|---|---|---|
| ≤ 15.42 % | 284.0 | 231.5 | 0.5894 | 0.29 - 1.21 |

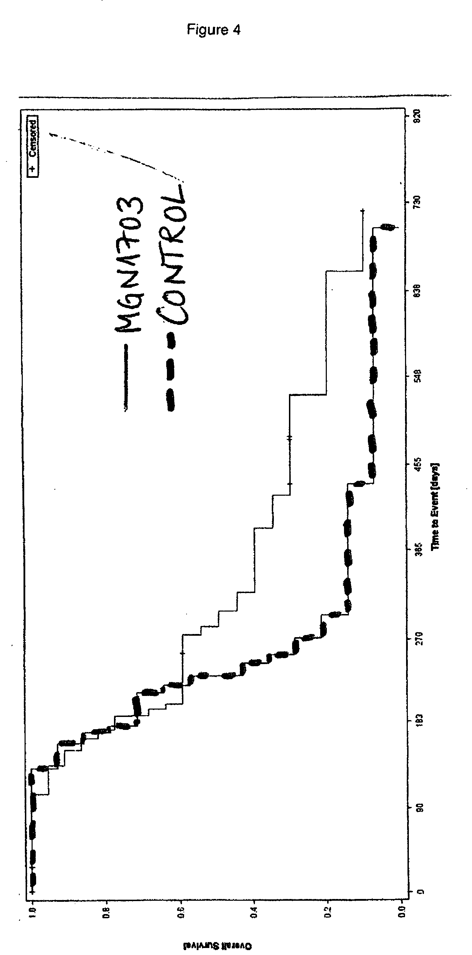

A Kaplan-Meier plot is shown in Figure 4 (patients treated with MGN1703: solid line; patients treated with control: dotted line). As can be seen in the plot and in Table 2, the patient subgroup with a ratio of activated B cells less than or equal to 15.42 % benefits from treatment with MGN1703 (median 284 days vs. 231.5 days (+52.5 days); HR 0.5894). Thus, the better survival probability of the patient subgroup with a ratio of activated B cells less than or equal to 15.42 % is related to the application of MGN1703. Patients with a ratio of activated B cells > 15.42 % did not benefit from treatment with MGN1703.

### Diagnosis of COPD

The following results were obtained for the patients that have been diagnosed with COPD (Table 3):

**Table 3: Results for patients diagnosed with COPD**

| Subgroup | MGN1703 Median (days) | Control Median (days) | Hazard ratio | 95 % Wald CI |
|---|---|---|---|---|
| Diagnosis of COPD | 316.0 | 246.0 | 0.5419 | 0.21 - 1.38 |

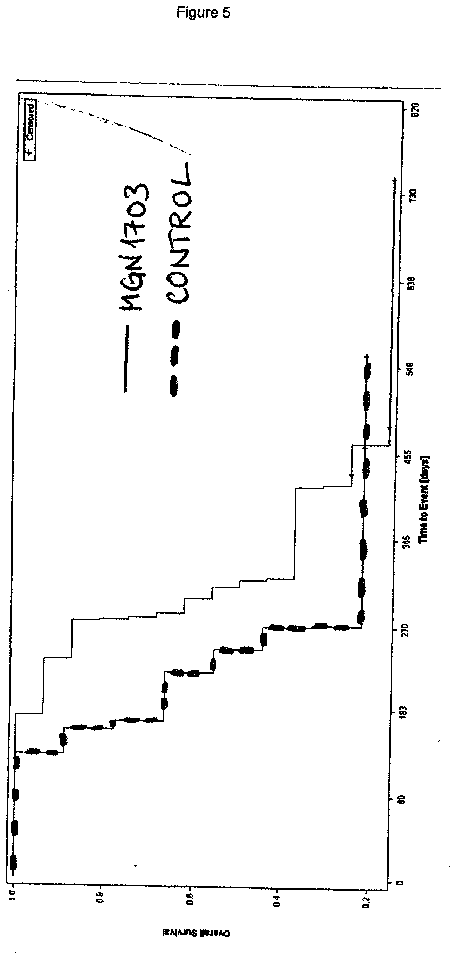

A Kaplan-Meier plot is shown in Figure 5 (patients treated with MGN1703: solid line; patients treated with control: dotted line). As can be seen in the plot and in Table 3, the patient subgroup diagnosed with COPD benefits from treatment with MGN1703 (median 316 days vs. 246 days (+70 days); HR 0.5419). Thus, the better survival probability of the patient subgroup with diagnosed COPD is related to the application of MGN1703. Patients who have not had COPD did not benefit from treatment with MGN1703.

The present invention provides new biomarkers for predicting a response to treatment with a TLR-9 agonist, in particular Lefitolimod, in a subject suffering from cancer, in particular from SCLC. The determination of the ratio of activated B cells and whether the patient has been diagnosed with COPD allows assessing the probability whether a patient is a responder to treatment with the TLR-9 agonist or not.

## Claims

1. A TLR-9 agonist, preferably Lefitolimod, for use in treating small cell lung cancer (SCLC) in a subject in need thereof, wherein the subject to be treated has one or more of the following characteristics:
i. a ratio of activated B cells with respect to the total number of CD19 positive cells of less than or equal to 20 %, preferably of less than or equal to 18 %, more preferably of less than or equal to 17 %, most preferably less than or equal to 15.4 %, and/or
ii. a diagnosed chronic obstructive pulmonary disease (COPD).

2. The TLR-9 agonist for use according to claim 1, wherein the activated B cells are CD86 positive.

3. The TLR-9 agonist for use according to claim 1 or 2, wherein the SCLC is limited SCLC or extensive SCLC, preferably extensive SCLC.

4. The TLR-9 agonist for use according to any of the preceding claims, wherein the subject has received a first cancer treatment before treatment with the TLR-9 agonist, in particular a platinum-based chemotherapeutic.

5. The TLR-9 agonist for use according to any of the preceding claims, wherein the TLR-9 agonist is used in a switch maintenance therapy.

6. The TLR-9 agonist for use according to any of the preceding claims, wherein the TLR-9 agonist, preferably Lefitolimod, is administered twice a week as switch maintenance therapy in a subject with extensive SCLC and wherein the subject achieved at least a partial response following first-line therapy with a platinum-based chemotherapeutic.

7. The TLR-9 agonist for use according to any of the preceding claims, wherein the TLR-9 agonist comprises a polydeoxyribonucleotide comprising at least one unmethylated CG dinucleotide, and wherein the at least one CG dinucleotide is part of a sequence N¹N²CGN³N⁴, wherein N¹N² is AA, TT, GG, GT, GA or AT and N³N⁴ is CT, TT or GG and C is deoxycytidine, G is deoxyguanosine, A is deoxyadenosine, and T is deoxythymidine.

8. The TLR-9 agonist for use according to claim 7, wherein the polydeoxyribonucleotide comprises at least one nucleotide in L-configuration.

9. The TLR-9 agonist for use according to claim 7 or 8, wherein the polydeoxyribonucleotide comprises a double-stranded stem and two single-stranded loops and forms the shape of a dumbbell.

10. The TLR-9 agonist for use according to claim 9, wherein the polydeoxyribonucleotide is covalently closed.

11. The TLR-9 agonist for use according to claim 9 or 10, wherein the at least one CG dinucleotide is located in one or each of the single-stranded loops.

12. The TLR-9 agonist for use according to any of claims 9 to 11, wherein the TLR-9 agonist consists of the sequence of SEQ ID NO: 3.

13. Method for predicting, whether a subject suffering from SCLC will respond to treatment with a TLR-9 agonist by determining before treatment with the TLR-9 agonist
i. a ratio of activated B cells with respect to the total number of CD19 positive cells, and/or
ii. whether the subject has been diagnosed with COPD.

14. Method for discriminating among subjects suffering from SCLC between a responder and a non-responder to treatment with a TLR-9 agonist by determining before treatment with the TLR-9 agonist
i. a ratio of activated B cells with respect to the total number of CD19 positive cells, and/or
ii. whether the subject has been diagnosed with COPD.

15. The method according to claim 14, wherein the responder has a better overall survival (OS) than the non-responder.

16. A pharmaceutical composition comprising 1 mg/ml to 30 mg/ml, preferably 10 mg/ml to 20 mg/ml, more preferably 15 mg/ml of Lefitolimod in PBS for use in treating SCLC, wherein the PBS has a pH of pH 6 to 8, in particular 7.0 to 7.5. and comprises
6 mg/ml to 12 mg/ml, preferably 8,8 mg/ml of sodium chloride,
0.1 mg/ml to 0.3 mg/ml, preferably 0.22 mg/ml of potassium chloride
0.1 mg/ml to 0.3 mg/ml, preferably 0.22 mg/ml of potassium dihydrogen phosphate and
1.0 mg/ml to 1.5 mg/ml, preferably 1.265 mg/ml of disodium hydrogen phosphate.

17. An activated B cell for use as a biomarker for predicting a response to treatment with a TLR-9 agonist, preferably Lefitolimod, in a subject suffering from cancer, in particular from SCLC.


SEQUENCE LISTING

<110>  Mologen AG

<120>  Biomarker for small cell lung cancer therapy

<130>  58 276 K

<160>  4

<170>  PatentIn version 3.5

<210>  1
<211>  58
<212>  DNA
<213>  Artificial Sequence

<220>
<223>  Partial sequence of Lefitolimod

<400>  1
cctaggggtt accaccttca ttggaaaacg ttcttcgggg cgttcttagg tggtaacc          58

<210>  2
<211>  58
<212>  DNA
<213>  Artificial Sequence

<220>
<223>  Partial sequence of dSLIM 2006

<400>  2
aggtggtaac ccctaggggt taccaccttc atcgtcgttt tgtcgttttg tcgttctt          58

<210>  3
<211>  116
<212>  DNA
<213>  Artificial Sequence

<220>
<223>  Complete sequence of Lefitolimod

<400>  3
aaaacgttct tcggggcgtt cttaggtggt aacccctagg ggttaccacc ttcattggaa          60

aacgttcttc ggggcgttct taggtggtaa cccctagggg ttaccacctt cattgg          116

<210>  4
<211>  116
<212>  DNA
<213>  Artificial Sequence

<220>
<223>  Complete sequence of dSLIM 2006

<400>  4
tcatcgtcgt tttgtcgttt tgtcgttctt aggtggtaac ccctaggggt taccaccttc          60

atcgtcgttt tgtcgttttg tcgttcttag gtggtaaccc ctaggggtta ccacct          116